(19) Europäisches Patentamt
European Patent Office
Office européen des brevets

(11) **EP 3 939 495 B1**

(12) **EUROPEAN PATENT SPECIFICATION**

(45) Date of publication and mention
of the grant of the patent:
**15.01.2025 Bulletin 2025/03**

(21) Application number: **20186267.9**

(22) Date of filing: **16.07.2020**

(51) International Patent Classification (IPC):
***A61B 5/00*** *(2006.01)*     ***H03F 1/34*** *(2006.01)*

(52) Cooperative Patent Classification (CPC):
**A61B 5/7214; A61B 5/30; A61B 5/7225;**
**A61B 5/725; H03F 1/34; H03F 3/45475;**
H03F 2200/261; H03F 2203/45138;
H03F 2203/45521

(54) **BIOSIGNAL MONITORING SYSTEM WITH MOTION ARTIFACT REDUCTION**

BIOSIGNALÜBERWACHUNGSSYSTEM MIT BEWEGUNGSARTEFAKTREDUKTION

SYSTÈME DE SURVEILLANCE DE BIOSIGNAL AVEC RÉDUCTION DES ARTÉFACTS DE MOUVEMENT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**19.01.2022 Bulletin 2022/03**

(73) Proprietors:
• **Imec VZW**
**3001 Leuven (BE)**
• **Stichting IMEC Nederland**
**5656 AE Eindhoven (NL)**

(72) Inventors:
• **Van Wegberg, Roland**
**3001 Leuven (BE)**
• **Van Helleputte, Nick**
**3001 Leuven (BE)**

(74) Representative: **Patent Department IMEC**
**IMEC vzw**
**Patent Department**
**Kapeldreef 75**
**3001 Leuven (BE)**

(56) References cited:
**EP-A1- 2 819 306     US-A1- 2013 116 577**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

# EP 3 939 495 B1

**Description**

<u>Technical field</u>

The present description is generally related to electronic systems for monitoring body signals and more specifically to biosignal monitoring systems with motion artifact reduction.

<u>Technical background</u>

Monitoring biopotential electrical signals, such as electrocardiogram (ECG), Electroencephalogram (EEG) or electromyogram (EMG) signals, over a certain time period may be used to evaluate or estimate a body condition of a person. When biopotential electrical signals are monitored using electrodes attached to the body, such as wet (gel) electrodes or dry electrodes, one of the major causes of motion artifact signals is the change in electrical properties of the skin-electrode/electrolyte interface that are known to have high correlation with motion/movement artifact, for example when running or stretching the skin.

A known technique for motion artifact removal is described in document "Real Time Digitally Assisted Analog Motion Artifact Reduction in Ambulatory ECG Monitoring System", by Sunyoung Kim et al., 34th Annual International Conference of the IEEE EMBS, San Diego, California USA, 28 August - 1 September 2012, wherein the electrode-skin impedance is measured and adopted as a reference signal for the digital adaptive filter, which then generates an estimated motion artifact signal that is subtracted from the measured biopotential signal in a programmable gain amplifier.

Another known technique for motion artifact removal by reducing the noise reference signal from an accelerometer in an adaptive filter is described in document "Using an Adaptive Filter to Remove ECG Motion Artifact Interference", by Li-Ming Bai et al., 2018 IEEE International Conference on Consumer Electronics-Taiwan (ICCE-TW).

US 2013/116577 discloses another technique requiring a digital motion artifact extraction module.

<u>Summary</u>

The invention is defined by the claims.

According to an example embodiment of the present disclosure, there is provided a biosignal monitoring system for reducing a motion artifact from at least one biopotential electrical signal input, the biosignal monitoring system comprising a signal processing module, a motion artifact extraction module and a subtraction module, wherein the motion artifact extraction module and the signal processing module receive the biopotential electrical signal input and the subtraction module receives both an extracted signal from an output of the motion artifact extraction module and a biopotential electrical signal from an output of the signal processing module; and the subtraction module is configured for subtracting the extracted signal from the biopotential electrical signal; wherein the motion artifact extraction module is implemented as analog domain electronic circuit and comprises a filter network, configured for attenuating differential mode signals of the biopotential electrical signal input from a first frequency, and passing a representation of the motion artifact signal from the biopotential electrical signal input up to a second frequency at the output of the motion artifact extraction module. According to an example, the system allows to advantageously extract the motion artifact signal more accurately and/or power efficiently. According to an example, the system is advantageous for single-end motion artifact reduction.

According to an example embodiment, the filter network comprises a first forward path amplifier having a first input connected to a first biopotential electrical signal input terminal, a second input and a output connected to the first output terminal of the motion artifact extraction module; and a second forward path amplifier having a first input connected to a second biopotential electrical signal input terminal, a second input and a output connected to the second output terminal of the motion artifact extraction module. The filter network further comprises a capacitor between the outputs of the first and second forward path amplifiers. According to an example, the motion artifact extraction module can be easily implemented. The filter network has a characteristic of a low pass filter for a differential mode signal and still pass a representation of a motion artifact signal.

According to an example embodiment, the capacitor in the filter network has a value between 100nF to 100uF. According to an example embodiment, the transconductance of the forward path amplifiers in the filter network has a value between 10 to 1000nS. According to an example embodiment, the forward path amplifiers are implemented as transconductance amplifiers or gm-stages.

According to an example embodiment, the motion artifact extraction module, the signal processing module and the subtraction module are implemented as analog domain electronic modules. According to still another example embodiment, the signal processing module and the subtraction module are implemented as an instrumentation amplifier; the biopotential electrical signal input is connected to both a first input of the instrumentation amplifier and an input of the motion artifact extraction module; the output of the motion artifact extraction module is connected to a second input of the instrumentation amplifier.

According to an example embodiment, the subtraction module is implemented as a digital domain electronic module. According to an example, the system is further able to reduce the motion artifact signal in a more controlled way in digital domain with adapted gains for different frequency.

According to still another example embodiment, the signal processing module is implemented as an instrumentation amplifier, the biosignal monitoring system further comprises: a first analog-to-digital converter connected to the output of the motion artifact extraction module and further connected to a first input of the subtraction module; and a second analog-to-digital connected to the output of the signal processing module and further connected to a second input of the subtraction module.

According to still another example embodiment, the motion artifact extraction module is integrated in an instrumentation amplifier further comprising a third forward path amplifier connected between one input terminal of the motion artifact extraction module and the biopotential electrical signal input terminal; and a fourth forward path amplifier connected between the other input terminal of the motion artifact extraction module and the biopotential electrical signal input terminal; a first analog-to-digital converter connected to the output of the motion artifact extraction module and further connected to a first input of the subtraction module; and a second analog-to-digital converter connected to the output of the signal processing module and further connected to a second input of the subtraction module.

According to an example embodiment, the subtraction module is implemented using a least mean squares algorithm.

According to an example embodiment, the biopotential electrical signal input is an ECG, EEG or EMG signal input.

According to an example embodiment, there is provided a microchip that comprises the described biosignal monitoring system. According to an example embodiment, a biomedical device may comprise the microchip or the described biosignal monitoring system.

According to an example embodiment, there is provided a method for reducing a motion artifact from at least one biopotential electrical signal input in the biosignal monitoring system, the method comprising the steps of extracting a representation of the motion artifact signal from the biopotential electrical signal input by attenuating a differential mode signal present in the biopotential electrical signal input from a first frequency and passing a representation of the motion artifact signal from the biopotential electrical signal input up to a second frequency; and subtracting the extracted representation of the motion artifact signal from the biopotential electrical signal input.

These as well other aspects, advantages, and alternatives will become apparent to those of ordinary skill in the art by reading the following detailed description, with reference where appropriate to the accompanying drawings.

Brief description of the drawings

The disclosure will be further elucidated by means of the following description and the appended figures. Various exemplary embodiments are described herein with reference to the following figures, wherein like numerals denote like entities. The figures described are schematic and non-limiting. Any reference signs in the claims shall not be construed as limiting the scope of the present disclosure. In the different figures, the same reference signs refer to the same or analogous elements.

**Figure 1** shows an example block diagram of a biosignal monitoring system for reducing motion artifacts from at least one biopotential electrical signal input.

**Figure 2a** shows an example schematic diagram of a motion artifact extraction module.

**Figure 2b** shows an example filter network circuit in the motion artifact extraction module presented in the schematic diagram of figure 2a.

**Figure 2c** shows an example of the gain at the output terminal of the motion artifact extraction module at different frequencies with respect to the differential mode signal or the motion artifact signal from the biopotential electrical signal input.

**Figure 3** shows a first example embodiment schematic circuit diagram of a biosignal monitoring system reducing motion artifacts from at least one biopotential electrical signal input.

**Figure 4** shows a second example embodiment schematic circuit diagram of a biosignal monitoring system reducing motion artifacts from at least one biopotential electrical signal input.

**Figure 5** shows a third example embodiment schematic diagram of a biosignal monitoring system reducing motion artifacts from at least one biopotential electrical signal input.

Detailed description

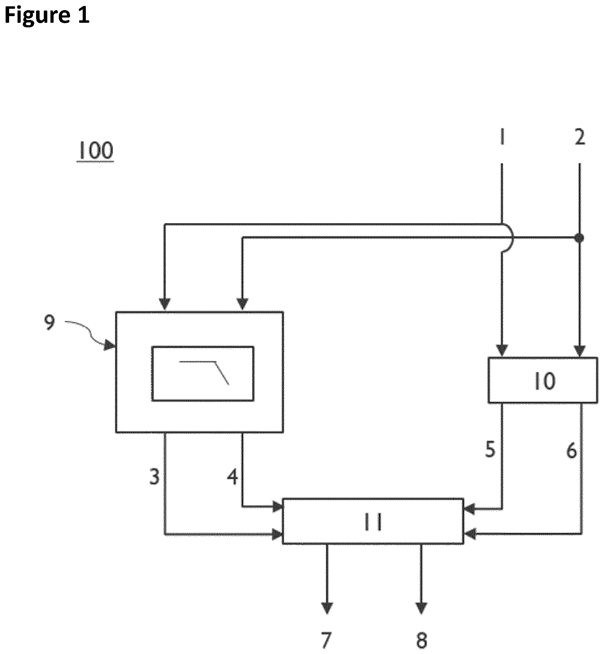

**Figure 1** shows a block diagram of a biosignal monitoring system 100 for reducing a motion artifact from at least one biopotential electrical signal input 1, 2, according to an example. The biosignal monitoring system 100 comprises three modules: a signal processing module 10, a motion artifact extraction module 9 including a filter network and a subtraction module 11.

The signal processing module 10 and the motion artifact extraction module 9 are configured for receiving the biopotential electrical signal input 1, 2 from corresponding electrodes, of a biomedical recording device, attached to a body. The biopotential electrical signal input 1, 2 comprises a differential mode signal and may also comprise a motion artifact signal. The motion artifact signal can be a single-end motion artifact signal which is the motion artifact signal from one terminal of the biopotential electrical signal input 1, 2 referring to the ground. The output 5, 6 of the signal processing module 10 comprises at least a differential mode signal and may comprise a motion artifact signal.

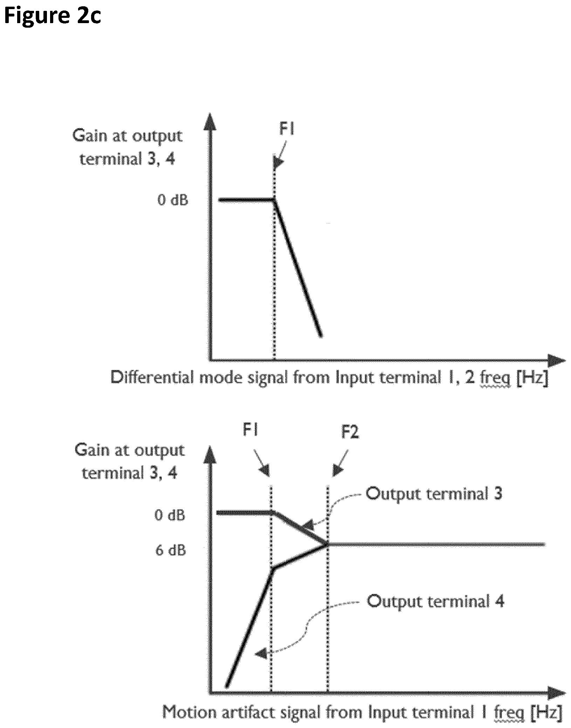

The motion artifact extraction module 9 is implemented as analog domain electronic circuit, and comprises a filter network which is configured for attenuating differential mode signals of the biopotential electrical signal input 1, 2 from a first frequency F1 (figure 2c), and passing a signal gain difference up to a second frequency F2 (figure 2c) at the output 3, 4 of the motion artifact extraction module 9, which is a representation of the motion artifact signal from the biopotential electrical signal input 1, 2. Thus, the motion artifact signal is extracted from the biopotential electrical signal input 1, 2 and provided at the output 3, 4 of the motion artifact extraction module 9. The output 3, 4 of the motion artifact extraction module 9 comprises at least a representation of the motion artifact signal and an attenuated differential mode signal.

The output 3, 4 of the motion artifact extraction module 9 is connected to a first input of the subtraction module 11, and the output 5, 6 of the signal processing module 10 is connected to a second input of the subtraction module 11. The subtraction module 11 is configured for subtracting the signals at the output 3, 4 of the motion artifact extraction module from the signals at the output 5, 6 of the signal processing module 10. The output 7, 8 of the subtraction module comprises at least a biopotential electrical signal with the motion artifact signal being reduced.

According to an example embodiment, the signal processing module 10 may adapt, process, filter and/or further amplify the biopotential electrical signal input 1, 2.

According to an example embodiment, the system 100 may be in a biomedical device, for example: an ECG, EEG or EMG monitoring device. Thus, the biopotential electrical signal input 1, 2 may be an ECG, EEG or EMG signal input.

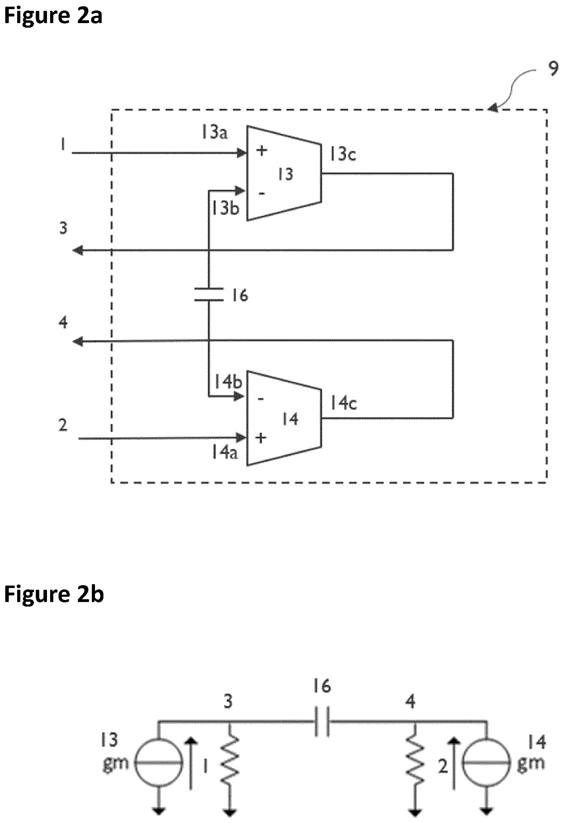

**Figure 2a** shows a schematic diagram of a motion artifact extraction module 9 including a filter network according to an example. The filter network comprises a first forward path amplifier 13, a second forward path amplifier 14 and a capacitor 16 between the outputs 13c, 14c of the forward path amplifiers 13, 14.

According to an example embodiment, the forward path amplifier 13, 14 each have a non-inverting input and an inverting input. The biopotential electrical signal input 1, 2 connected to the non-inverting inputs 13a, 14a. The output 13c of the first forward path amplifier 13 is connected to the inverting input 13b and the output terminal 3 of the motion artifact extraction module 9; the output 14c of the second forward path amplifier 14 is connected to the inverting input 14b and the output terminal 4 of the motion artifact extraction module 9. The capacitor 16 is connected between outputs 13c, 14c, which is also the inverting inputs 13b, 14b, of the forward path amplifiers 13, 14. Thus, the forward path amplifier 13 and the capacitor 16 function as a gm-C low pass filter for the signal at the biopotential electrical signal input 1, and the forward path amplifier 14 and the capacitor 16 function as a gm-C low pass filter for the signal at the biopotential electrical signal input 2.

**Figure 2b** shows an example filter network circuit in the motion artifact extraction module presented in the schematic diagram of in figure 2a. The output impedances are also shown in the circuit (not shown in the figure 2a).

According to an example embodiment, the transconductance is the same for the forward path amplifier 13 and 14. The output impedance R is assumed to be the same for the output terminals 3 and 4.

The filter network has a low pass filter characteristic with a cut-off frequency at a first frequency F1. The transfer function in respect of the differential mode signal from the biopotential electrical signal input 1, 2 to output 3, 4 is:

$$\frac{Vout_{DM}}{Vin_{DM}} = \frac{g_m * R}{(1 + g_m * R) + s * R * 2C} \ and \ pole \ s = \frac{-g_m}{2C}$$

Wherein $Vout_{DM}$ is the output voltage of the filter network in respect to the differential mode signal at the biopotential electrical signal input 1, 2, $Vin_{DM}$ is the input voltage of the filter network in respect to the differential mode signal at the biopotential electrical signal input 1, 2, gm is the transconductance gain of the forward path amplifiers and C is the value of the capacitor 16, R is the output impedance of output terminals 3, 4 (shown in the figure 2b).

According to an example embodiment, the motion artifact signal is a single-end motion artifact signal at the biopotential electrical signal 1. The filter network has different gain responses at the output terminal 3 and 4 in respect to the motion artifact signal. The transfer function in respect of the motion artifact signal from the biopotential electrical signal 1 to output terminal 3 is (it is applicable to the motion artifact signal from the biopotential electrical signal 2 to output terminal 4):

$$\frac{VoutP_{MA}}{VinP_{MA}} = \frac{g_m * R * (1 + g_m * R + s * R * C)}{(1 + g_m{}^2 * R^2) + s * g_m * R^2 * 2C} \quad and \quad pole \; s = \frac{-g_m}{2C}, zero \; s = \frac{-g_m}{C}$$

Wherein $VoutP_{MA}$ is the output voltage at output terminal 3 in respect to the motion artifact signal at the biopotential electrical signal input 1, $VinP_{MA}$ is the input voltage of the motion artifact signal at the biopotential electrical signal input 1.

And the transfer function of the motion artifact signal from biopotential electrical signal 1 to output terminal 4 is (it is applicable to the motion artifact signal from biopotential electrical signal 2 to output terminal 3):

$$\frac{VoutN_{MA}}{VinP_{MA}} = \frac{s * g_m * R^2 * 2C}{(1 + g_m{}^2 * R^2) + s * g_m * R^2 * 2C} \quad and \quad pole \; s = \frac{-g_m}{2C}, zero \; s = 0$$

Wherein $VoutN_{MA}$ is the output voltage of output 4 in respect to the motion artifact signal at the biopotential electrical signal input 1.

**Figure 2c** shows an example of the gain at output terminal 3, 4 corresponding to different frequencies with respect to the differential mode signal or the single-end motion artifact signal of the biopotential electrical signal input terminal 1, 2.

As indicated in the transfer functions, the first frequency F1 is equal to gm/2C for the differential signal and the motion artifact signal. The signals at output terminal 3 and 4 of the motion artifact extraction module 9 have different gain responses in respect of the motion artifact signal, and the gain difference is the representation of the motion artifact signal. The gain at the output terminal 3 and 4 of the motion artifact extraction module 9 finally reaches the same value at the second frequency F2, which is equal to gm/C, and remains the same after the second frequency F2. The first frequency F1 and the second frequency F2 therefore cannot be designed independently of each other. By properly designing capacitor 16 and the forward path transistors of the feedback network filter, the first frequency F1 and the second frequency F2 can be tuned accordingly. The output 3, 4 includes a representation of the motion artifact signals up to the second frequency F2 and an attenuated low-frequency differential signal from the first frequency F1.

According to an example, motion artifact signals are in the frequency range of up to 4 Hz, with the most artifacts in the frequency range around 2 Hz. According to an example embodiment, if the first frequency F1 is tuned to 0.5 Hz, the second frequency F2 is accordingly equal to 1 Hz, the output 3, 4 of the motion artifact extraction module 9 comprises the differential mode signal attenuated from 0.5 Hz and a representation of the motion artifact signal up to 1 Hz. According to an example embodiment, if the first frequency F1 is tuned to 2 Hz, the second frequency F2 is accordingly equal to 4 Hz, the output 3, 4 of the motion artifact extraction module 9 comprises the differential mode signal attenuated from 2 Hz and a representation of the motion artifact signal up to 4 Hz.

According to an example embodiment, the capacitor 16 may have a value between 100nF to 100 uF. According to an example embodiment, the transconductance amplifiers may have a transconductance of between 10 to 1000 nS (nano Siemens).

According to an example embodiment, the forward path amplifiers 13, 14 are transconductance amplifiers or gm-stages.

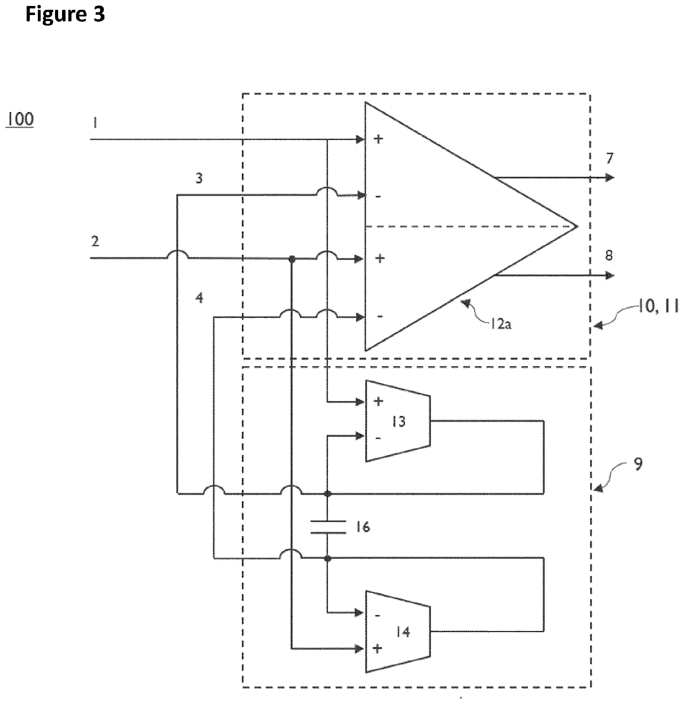

**Figure 3** shows a first schematic circuit diagram of a biosignal monitoring system 100 for reducing a motion artifact from at least one biopotential electrical signal input 1, 2 according to an example embodiment. The system 100 comprises a biopotential electrical signal input 1, 2, an instrumentation amplifier 12a, a signal processing module 10, a motion artifact extraction module 9 including a filter network, and a subtraction module 11.

According to an example embodiment, the motion artifact extraction module 9, the signal processing module 10 and the subtraction module 11 in system 100 are implemented as analog domain electronic circuits.

According to an example embodiment, both the signal processing module 10 and the subtraction module 11 are implemented inside the instrumentation amplifier 12a. Thus, the input of the instrumentation amplifier 12a is the input 1, 2 of the signal processing module 10; the output of the instrumentation amplifier 12a is the output 7, 8 of the subtraction module 11 and the output 5, 6 of the signal processing module 10 becomes an internal output inside the instrumentation amplifier 12a (not shown in figure 3). The biopotential electrical signal input 1, 2 is connected to both a first input of the instrumentation amplifier 12a and the input of the motion artifact extraction module 9; the output 3, 4 of the motion artifact extraction module 9 is connected to a second input of the instrumentation amplifier 12a. The output 3, 4 of the motion artifact extraction module 9 comprises a motion artifact signal. The output 7, 8 of the instrumentation amplifier 12a comprises the biopotential electrical signal with the motion artifact signal being reduced. The instrumentation amplifier 12a may be implemented as two differential amplifiers. According to an example embodiment, the instrumentation amplifier 12a may comprise a gain adjustment to the output 3, 4 of the motion artifact extraction module 9.

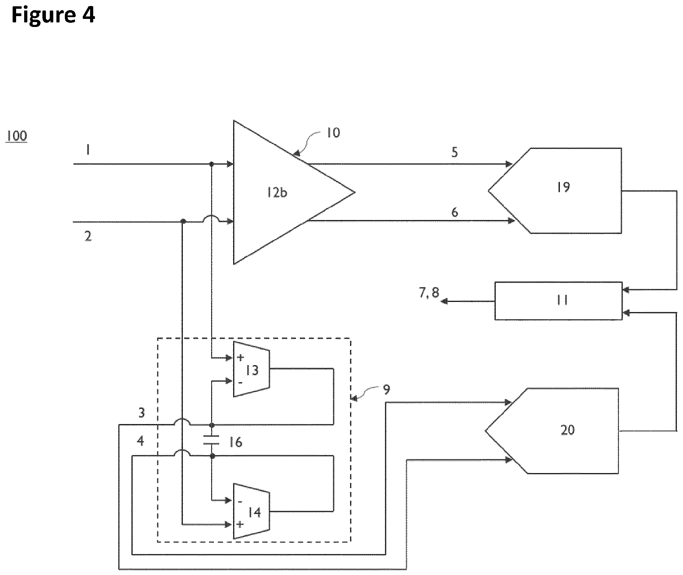

**Figure 4** shows a second schematic circuit diagram of a biosignal monitoring system 100 for reducing a motion artifact from at least one biopotential electrical signal input 1, 2 according to an example embodiment. The system 100 comprises a biopotential electrical signal input 1, 2, an instrumentation amplifier 12b, a motion artifact module 9 including a filter network connected to a first ADC 20, a signal processing module 10 connected to a second ADC (Analog-to-Digital Converter) 19, and a subtraction module 11.

According to an example embodiment, the subtraction module 11 is implemented as a digital domain electronic circuit.

According to an example embodiment, the signal processing module 10 is implemented as an instrumentation amplifier 12b. The biopotential electrical signal input 1, 2 is connected to the motion artifact extraction module 9. The output 3, 4 of the motion artifact extraction module 9 is connected to the input of the first ADC 20. The output of the first ADC 20 is connected to a first input of the subtraction module 11. The biopotential electrical signal input 1, 2 is also connected to the instrumentation amplifier 12b. The output of the instrumentation amplifier 12b, which is the output 5, 6 of the signal processing module 10, is connected to the input of the second ADC 19. The output of the second ADC 19 is connected to a second input of the subtraction module 11. The output of the second ADC 19 comprises a digitized differential mode signal and a digitized motion artifact signal. The output of the first ADC 20 comprises a digitized motion artifact signal. The output 3, 4 of the motion artifact extraction module 9 will be subtracted from the output 5, 6 of the signal processing module 10 in the subtraction module 11. The output 7, 8 of the subtraction module 11 comprises the biopotential electrical signal with the motion artifact signal being reduced.

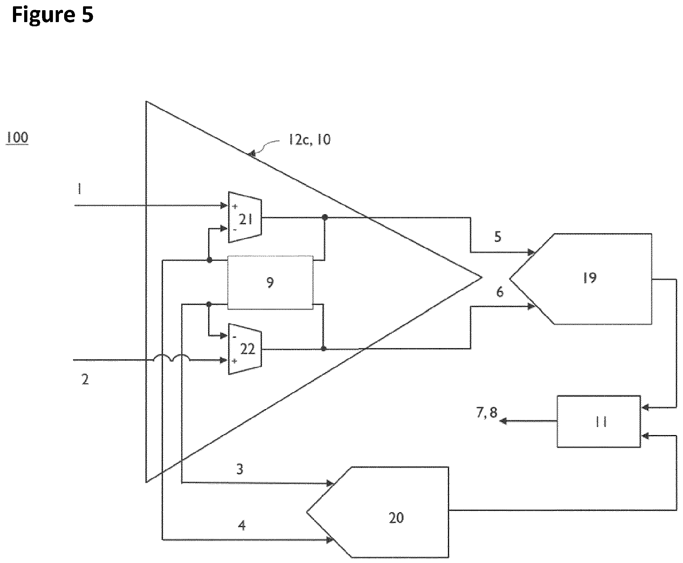

**Figure 5** shows a third schematic circuit diagram of a biosignal monitoring system 100 for reducing a motion artifact from at least one biopotential electrical signal input 1, 2 according to an example embodiment. The system 100 comprises a biopotential electrical signal input 1, 2, an instrumentation amplifier 12c, a third forward path amplifiers 21, a fourth forward path amplifier 22, a first ADC 20 and a second ADC 19, a motion artifact extraction module 9, a signal processing module 10 and a subtraction module 11. The signal processing module 10 is implemented as an instrumentation amplifier 12c. The motion artifact extraction module 9 is integrated in the instrumentation amplifier 12c. The subtraction module 11 is implemented as a digital domain electronic module.

According to an example embodiment, the subtraction module 11 is implemented using a least mean squares algorithm. According to an example embodiment, the subtraction module 11 may comprise a gain adjustment function.

According to an example embodiment, the biopotential electrical signal input 1, 2 is connected to the input of the instrumentation amplifier 12c which is the input of the signal processing module 10. The biopotential electrical signal input terminal 1 is connected to the third forward path amplifier 21 and the output of the third forward path amplifier 21 is connected to one input of the motion artifact extraction module 9. The biopotential electrical signal input terminal 2 is connected to the fourth forward path amplifier 22 and the output of the fourth forward path amplifier 22 is connected to the other input of the motion artifact extraction module 9. The output of the forward path amplifier 21, 22 comprises the biopotential electrical signal input 1, 2. The output of 3, 4 of the motion artifact extraction module 9 is further connected to the first ADC 20. The output of the first ADC 20 comprises a digitized motion artifact signal. The output of the instrumentation amplifier 12c, which is the output 5, 6 of the signal processing module 10, is further connected to the second ADC 19. The output of the second ADC 19 comprises a digitized differential mode signal and a digitized motion artifact signal. The output of the first ADC 20 is connected to a first input of the subtraction module 11; the output the second ADC 19 is connected to a second input of the subtraction module 11. The output 7, 8 of the subtraction module 11 comprises a digitized biopotential electrical signal with the motion artifact signal being much reduced.

According to an example embodiment, the input of the motion artifact module 9 may be between any intermediate nodes in the instrumentation amplifier 12c where the input of the motion artifact module 9 comprises a biopotential electrical signal having at least a differential mode signal and a motion artifact signal.

According to an example embodiment, the two forward path amplifiers 21, 22 may be opamps with closed loop gain control, or instrumentation amplifiers.

According to an example embodiment, the subtraction module 11 may comprise a gain adjustment function. The representation of the motion artifact signal extracted from the motion artifact extraction module 9 may be an attenuated motion artifact signal and the subtraction module 11 may be configured to adjust the gain of the motion artifact signal before it is subtracted from the output 5, 6 of the signal processing module 10.

According to an example embodiment, the subtraction module 11 is implemented using a least mean squares (LMS) algorithm.

According to an example embodiment, a method for reducing a motion artifact from at least one biopotential electrical signal input 1, 2 in the biosignal monitoring system comprises the steps of: extracting a representation of the motion artifact signal from the biopotential electrical signal input 1, 2 by attenuating a differential mode signal present in the biopotential electrical signal input 1, 2 from a first frequency F1 and passing a representation of the motion artifact signal from the biopotential electrical signal input 1, 2 up to a second frequency F2; and subtracting the extracted representation of the motion artifact signal from the biopotential electrical signal input 1, 2.

**Claims**

1. A biosignal monitoring system (100) for reducing a motion artifact from at least one biopotential electrical signal input (1, 2), the biosignal monitoring system comprising a signal processing module (10), a motion artifact extraction module (9) and a subtraction module (11), wherein the motion artifact extraction module (9) and the signal processing module (10) receive the biopotential electrical signal input (1, 2) and the subtraction module (11) receives both an extracted signal from an output (3, 4) of the motion artifact extraction module (9) and a biopotential electrical signal from an output (5, 6) of the signal processing module (10); and the subtraction module (11) is configured for subtracting the extracted signal from the biopotential electrical signal;
   **characterized in that**:
   the motion artifact extraction module (9) is implemented as analog domain electronic circuit and comprises a filter network, configured for attenuating differential mode signals of the biopotential electrical signal input (1, 2) from a first frequency (F1), and passing a representation of the motion artifact signal from the biopotential electrical signal input (1, 2) up to a second frequency (F2) at the output (3, 4) of the motion artifact extraction module (9).

2. A biosignal monitoring system according to claim 1, wherein the filter network comprises a first forward path amplifier (13) having a first input (13a) connected to a first biopotential electrical signal input terminal (1), a second input (13b) and a output (13c) connected to the first output terminal (3) of the motion artifact extraction module (9); and a second forward path amplifier (14) having a first input (14a) connected to a second biopotential electrical signal input terminal (2), a second input (14b) and a output (14c) connected to the second output terminal (4) of the motion artifact extraction module (9); and a capacitor (16) between the output (13c) of the first forward path amplifier (13) and the output (14c) of the second forward path amplifier (14).

3. A biosignal monitoring system according to claim 2, wherein the capacitor 16 has a value between 100nF to 100 uF.

4. A biosignal monitoring system according to any of claims 2 to 3, wherein the transconductance of the forward path amplifier (13, 14) has a value between 10 to 1000 nS.

5. A biosignal monitoring system according to any of claims 2 to 4, wherein the forward path amplifiers (13, 14) are implemented as transconductance amplifiers or gm-stages.

6. A biosignal monitoring system according to any of the preceding claims, wherein the motion artifact extraction module (9), the signal processing module (10) and the subtraction module (11) are implemented as analog domain electronic modules.

7. A biosignal monitoring system according to claim 6, wherein the signal processing module (10) and the subtraction module (11) are implemented as an instrumentation amplifier (12a); the biopotential electrical signal input (1, 2) is connected to both a first input of the instrumentation amplifier (12a) and an input of the motion artifact extraction module (9); the output (3, 4) of the motion artifact extraction module (9) is connected to a second input of the instrumentation amplifier (12a).

8. A biosignal monitoring system according to any of the claims 1 to 5, wherein the subtraction module (11) is implemented as a digital domain electronic module.

9. A biosignal monitoring system according to claim 8 wherein the signal processing module (10) is implemented as an instrumentation amplifier (12b), the biosignal monitoring system further comprises: a first analog-to-digital converter (20) connected to the output (3, 4) of the motion artifact extraction module (9) and further connected to a first input of the subtraction module (11); and a second analog-to-digital connected to (19) the output (5, 6) of the signal processing module (10) and further connected to a second input of the subtraction module (11).

10. A biosignal monitoring system according to claim 8, wherein the motion artifact extraction module (9) is integrated in an instrumentation amplifier (12c) further comprising a third forward path amplifier (21) connected between one input terminal of the motion artifact extraction module (9) and the biopotential electrical signal input terminal (1); and a fourth forward path amplifier (22) connected between the other input terminal of the motion artifact extraction module (9) and the biopotential electrical signal input terminal (2); a first analog-to-digital converter (20) connected to the output (3, 4)

of the motion artifact extraction module (9) and further connected to a first input of the subtraction module (11); and a second analog-to-digital converter (19) connected to the output (5, 6) of the signal processing module (10) and further connected to a second input of the subtraction module (11).

11. A biosignal monitoring system according to any of the claims 8 to 10, wherein the subtraction module (11) is implemented using a least mean squares algorithm.

12. A biosignal monitoring system according to any preceding claims, wherein the biopotential electrical signal input (1 ,2) is an ECG, EEG or EMG signal input.

13. A microchip comprising a biosignal monitoring system according to any preceding claims.

14. A biomedical device comprising a microchip according to claim 13 or a biosignal monitoring system according to any of the claims 1 to 12.

15. A method for reducing a motion artifact from at least one biopotential electrical signal input (1, 2) in the biosignal monitoring system according to any of claims 1 to 12, the method comprising steps of:

- extracting a representation of the motion artifact signal from the biopotential electrical signal input (1, 2) by attenuating a differential mode signal present in the biopotential electrical signal input (1, 2) from a first frequency (F1) and passing a representation of the motion artifact signal from the biopotential electrical signal input (1, 2) up to a second frequency (F2);
- subtracting the extracted representation of the motion artifact signal from the biopotential electrical signal input (1, 2).

**Patentansprüche**

1. Biosignal-Überwachungssystem (100) zum Reduzieren eines Bewegungsartefakts von mindestens einem biopotentiellen elektrischen Signaleingang (1, 2), wobei das Biosignal-Überwachungssystem ein Signalverarbeitungsmodul (10), ein Bewegungsartfakt-Extraktionsmodul (9) und ein Subtraktionsmodul (11) umfasst, wobei das Bewegungsartfakt-Extraktionsmodul (9) und das Signalverarbeitungsmodul (10) den biopotentiellen elektrischen Signaleingang (1, 2) empfangen und das Subtraktionsmodul (11) sowohl ein extrahiertes Signal von einem Ausgang (3, 4) des Bewegungsartfakt-Extraktionsmoduls (9) als auch ein biopotentielles elektrisches Signal von einem Ausgang (5, 6) des Signalverarbeitungsmoduls (10) empfängt; und das Subtraktionsmodul (11) zum Subtrahieren des aus dem biopotentiellen elektrischen Signal extrahierten Signals konfiguriert ist;
**dadurch gekennzeichnet, dass**:
das Bewegungsartfakt-Extraktionsmodul (9) als elektronische Schaltung im analogen Bereich implementiert ist und ein Filternetzwerk umfasst, das zum Dämpfen von Differenzialmodussignalen des biopotentiellen elektrischen Signaleingangs (1, 2) ab einer ersten Frequenz (F1) und zum Weiterleiten einer Darstellung des Bewegungsartfakt-Signals vom biopotentiellen elektrischen Signaleingang (1, 2) bis zu einer zweiten Frequenz (F2) am Ausgang (3, 4) des Bewegungsartfakt-Extraktionsmoduls (9) konfiguriert ist.

2. Biosignal-Überwachungssystem nach Anspruch 1, wobei das Filternetzwerk einen ersten Vorwärtspfadverstärker (13) umfasst, der einen ersten Eingang (13a) aufweist, der mit einem ersten biopotentiellen elektrischen Signaleingangsanschluss (1) verbunden ist, einem zweiten Eingang (13b) und einem Ausgang (13c), der mit dem ersten Ausgangsanschluss (3) des Bewegungsartfakt-Extraktionsmoduls (9) verbunden ist, und einen zweiten Vorwärtspfadverstärker (14), der einen ersten Eingang (14a) aufweist, der mit einem zweiten biopotentiellen elektrischen Signaleingangsanschluss (2) verbunden ist, einem zweiten Eingang (14b) und einem Ausgang (14c), der mit dem zweiten Ausgangsanschluss (4) des Bewegungsartfakt-Extraktionsmoduls (9) verbunden ist, und einen Kondensator (16) zwischen dem Ausgang (13c) des ersten Vorwärtspfadverstärkers (13) und dem Ausgang (14c) des zweiten Vorwärtspfadverstärkers (14).

3. Biosignal-Überwachungssystem nach Anspruch 2, wobei der Kondensator (16) einen Wert zwischen 100 nF und 100 uF aufweist.

4. Biosignal-Überwachungssystem nach einem der Ansprüche 2 bis 3, wobei die Steilheit des Vorwärtspfadverstärkers (13, 14) einen Wert zwischen 10 und 1000 nS aufweist.

**5.** Biosignal-Überwachungssystem nach einem der Ansprüche 2 bis 4, wobei die Vorwärtspfadverstärker (13,14) als Steilheitsverstärker oder GM-Stufen implementiert sind.

**6.** Biosignal-Überwachungssystem nach einem der vorstehenden Ansprüche, wobei das Bewegungsartfakt-Extraktionsmodul (9), das Signalverarbeitungsmodul (10) und das Subtraktionsmodul (11) als elektronische Module im analogen Bereich implementiert sind.

**7.** Biosignal-Überwachungssystem nach Anspruch 6, wobei das Signalverarbeitungsmodul (10) und das Subtraktionsmodul (11) als Differenzverstärker (12a) implementiert sind; der biopotenzielle elektrische Signaleingang (1, 2) sowohl mit einem ersten Eingang des Differenzverstärkers (12a) als auch mit einem Eingang des Bewegungsartfakt-Extraktionsmoduls (9) verbunden ist; der Ausgang (3, 4) des Bewegungsartfakt-Extraktionsmoduls (9) mit einem zweiten Eingang des Differenzverstärkers (12a) verbunden ist.

**8.** Biosignal-Überwachungssystem nach einem der Ansprüche 1 bis 5, wobei das Subtraktionsmodul (11) als elektronisches Modul im digitalen Bereich implementiert ist.

**9.** Biosignal-Überwachungssystem nach Anspruch 8, wobei das Signalverarbeitungsmodul (10) als Differenzverstärker (12b) implementiert ist und das Biosignal-Überwachungssystem weiter Folgendes umfasst: einen ersten Analog-Digital-Umsetzer (20), der mit dem Ausgang (3, 4) des Bewegungsartfakt-Extraktionsmoduls (9) verbunden ist und weiter mit einem ersten Eingang des Subtraktionsmoduls (11) verbunden ist; und einen zweiten Analog-Digital-Umsetzer (19), der mit dem Ausgang (5, 6) des Signalverarbeitungsmoduls (10) verbunden ist und weiter mit einem zweiten Eingang des Subtraktionsmoduls (11) verbunden ist.

**10.** Biosignal-Überwachungssystem nach Anspruch 8, wobei das Bewegungsartfakt-Extraktionsmodul (9) in einen Differenzverstärker (12c) integriert ist, der weiter einen dritten Vorwärtspfadverstärker (21) umfasst, der zwischen einem Eingangsanschluss des Bewegungsartfakt-Extraktionsmoduls (9) und dem biopotenziellen elektrischen Signaleingangsanschluss (1) verbunden ist; und einen vierten Vorwärtspfadverstärker (22), der zwischen dem anderen Eingangsanschluss des Bewegungsartfakt-Extraktionsmoduls (9) und dem biopotentiellen elektrischen Signaleingangsanschluss (2) verbunden ist; einen ersten Analog-Digital-Umsetzer (20), der mit dem Ausgang (3, 4) des Bewegungsartfakt-Extraktionsmoduls (9) und weiter mit einem ersten Eingang des Subtraktionsmoduls (11) verbunden ist; und einen zweiten Analog-Digital-Umsetzer (19), der mit dem Ausgang (5, 6) des Signalverarbeitungsmoduls (10) und weiter mit einem zweiten Eingang des Subtraktionsmoduls (11) verbunden ist.

**11.** Biosignal-Überwachungssystem nach einem der Ansprüche 8 bis 10, wobei das Subtraktionsmodul (11) unter Verwendung eines Algorithmus der kleinsten Fehlerquadrate implementiert ist.

**12.** Biosignal-Überwachungssystem nach einem der vorstehenden Ansprüche, wobei der biopotenzielle elektrische Signaleingang (1,2) ein EKG-, EEG- oder EMG-Signaleingang ist.

**13.** Mikrochip, der ein Biosignal-Überwachungssystem nach einem der vorstehenden Ansprüche umfasst.

**14.** Biomedizinische Vorrichtung, die einen Mikrochip nach Anspruch 13 oder ein Biosignal-Überwachungssystem nach einem der Ansprüche 1 bis 12 umfasst.

**15.** Verfahren zum Reduzieren eines Bewegungsartefakts von mindestens einem biopotentiellen elektrischen Signaleingang (1, 2) im Biosignal-Überwachungssystem nach einem der Ansprüche 1 bis 12, wobei das Verfahren folgende Schritte umfasst:

- Extrahieren einer Darstellung des Bewegungsartfakt-Signals aus dem biopotentiellen elektrischen Signaleingang (1, 2) durch Dämpfen eines im biopotentiellen elektrischen Signaleingang (1, 2) vorhandenen Differenzialmodussignals von einer ersten Frequenz (F1) und Weiterleiten einer Darstellung des Bewegungsartfakt-Signals von dem biopotentiellen elektrischen Signaleingang (1, 2) bis zu einer zweiten Frequenz (F2);
- Subtrahieren der extrahierten Darstellung des Bewegungsartfakt-Signals vom biopotentiellen elektrischen Signaleingang (1, 2).

**Revendications**

1. Système de surveillance de biosignal (100) pour réduire un artefact de mouvement à partir d'au moins une entrée de signal électrique biopotentiel (1, 2), le système de surveillance de biosignal comprenant un module de traitement de signal (10), un module d'extraction d'artefact de mouvement (9) et un module de soustraction (11), dans lequel le module d'extraction d'artefact de mouvement (9) et le module de traitement de signal (10) reçoit l'entrée de signal électrique biopotentiel (1, 2) et le module de soustraction (11) reçoit à la fois un signal extrait à partir d'une sortie (3, 4) du module d'extraction d'artefact de mouvement (9) et un signal électrique biopotentiel à partir d'une sortie (5, 6) du module de traitement de signal (10) ; et le module de soustraction (11) est configuré pour soustraire le signal extrait à partir du signal électrique biopotentiel ;
**caractérisé en ce que** :
le module d'extraction d'artefact de mouvement (9) est mis en œuvre en tant que circuit électronique de domaine analogique et comprend un réseau de filtre, configuré pour atténuer des signaux de mode différentiel de l'entrée de signal électrique biopotentiel (1, 2) à partir d'une première fréquence (F1), et faire passer une représentation du signal d'artefact de mouvement à partir de l'entrée de signal électrique biopotentiel (1, 2) jusqu'à une deuxième fréquence (F2) à la sortie (3, 4) du module d'extraction d'artefact de mouvement (9).

2. Système de surveillance de biosignal selon la revendication 1, dans lequel le réseau de filtre comprend un premier amplificateur de chaîne d'action (13) présentant une première entrée (13a) connectée à une première borne d'entrée de signal électrique biopotentiel (1), une seconde entrée (13b) et une sortie (13c) connectée à la première borne de sortie (3) du module d'extraction d'artefact de mouvement (9) ; et un deuxième amplificateur de chaîne d'action (14) présentant une première entrée (14a) connectée à une deuxième borne d'entrée de signal électrique biopotentiel (2), une deuxième entrée (14b) et une sortie (14c) connectée à la deuxième borne de sortie (4) du module d'extraction d'artefact de mouvement (9) ; et un condensateur (16) entre la sortie (13c) du premier amplificateur de chaîne d'action (13) et la sortie (14c) du deuxième amplificateur de chaîne d'action (14).

3. Système de surveillance de biosignal selon la revendication 2, dans lequel le condensateur 16 présente une valeur entre 100 nF et 100 uF.

4. Système de surveillance de biosignal selon l'une quelconque des revendications 2 à 3, dans lequel la transconductance de l'amplificateur de chaîne d'action (13, 14) présente une valeur entre 10 et 1 000 nS.

5. Système de surveillance de biosignal selon l'une quelconque des revendications 2 à 4, dans lequel les amplificateurs de chaîne d'action (13, 14) sont mis en œuvre en tant qu'amplificateurs de transconductance ou d'étages gm.

6. Système de surveillance de biosignal selon l'une quelconque des revendications précédentes, dans lequel le module d'extraction d'artefact de mouvement (9), le module de traitement de signal (10) et le module de soustraction (11) sont mis en œuvre en tant que modules électroniques de domaine analogique.

7. Système de surveillance de biosignal selon la revendication 6, dans lequel le module de traitement de signal (10) et le module de soustraction (11) sont mis en œuvre en tant qu'amplificateur d'instrumentation (12a) ; l'entrée de signal électrique biopotentiel (1, 2) est connectée à la fois à une première entrée de l'amplificateur d'instrumentation (12a) et à une entrée du module d'extraction d'artefact de mouvement (9) ; la sortie (3, 4) du module d'extraction d'artefact de mouvement (9) est connectée à une deuxième entrée de l'amplificateur d'instrumentation (12a).

8. Système de surveillance de biosignal selon l'une quelconque des revendications 1 à 5, dans lequel le module de soustraction (11) est mis en œuvre en tant que module électronique de domaine numérique.

9. Système de surveillance de biosignal selon la revendication 8, dans lequel le module de traitement de signal (10) est mis en œuvre en tant qu'amplificateur d'instrumentation (12b), le système de surveillance de biosignal comprenant en outre : un premier convertisseur analogique-numérique (20) connecté à la sortie (3, 4) du module d'extraction d'artefact de mouvement (9) et connecté en outre à une première entrée du module de soustraction (11) ; et un deuxième convertisseur analogique-numérique (19) connecté à la sortie (5, 6) du module de traitement de signal (10) et connecté en outre à une deuxième entrée du module de soustraction (11).

10. Système de surveillance de biosignal selon la revendication 8, dans lequel le module d'extraction d'artefact de mouvement (9) est intégré dans un amplificateur d'instrumentation (12c) comprenant en outre un troisième amplificateur de chaîne d'action (21) connecté entre une borne d'entrée du module d'extraction d'artefact de mouvement (9) et la borne d'entrée de signal électrique biopotentiel (1) ; et un quatrième amplificateur de chaîne d'action (22) connecté entre l'autre borne d'entrée du module d'extraction d'artefact de mouvement (9) et la borne d'entrée de signal électrique biopotentiel (2) ; un premier convertisseur analogique-numérique (20) connecté à la sortie (3, 4) du module d'extraction d'artefact de mouvement (9) et connecté en outre à une première entrée du module de soustraction (11) ; et un deuxième convertisseur analogique-numérique (19) connecté à la sortie (5, 6) du module de traitement de signal (10) et connecté en outre à une deuxième entrée du module de soustraction (11).

11. Système de surveillance de biosignal selon l'une quelconque des revendications 8 à 10, dans lequel le module de soustraction (11) est mis en œuvre en utilisant un algorithme des moindres carrés moyens.

12. Système de surveillance de biosignal selon l'une quelconque des revendications précédentes, dans lequel l'entrée de signal électrique biopotentiel (1, 2) est une entrée de signal ECG, EEG ou EMG.

13. Micropuce comprenant un système de surveillance de biosignal selon l'une quelconque des revendications précédentes.

14. Dispositif biomédical comprenant une micropuce selon la revendication 13 ou un système de surveillance de biosignal selon l'une quelconque des revendications 1 à 12.

15. Procédé de réduction d'un artefact de mouvement à partir d'au moins une entrée de signal électrique biopotentiel (1, 2) dans le système de surveillance de biosignal selon l'une quelconque des revendications 1 à 12, le procédé comprenant les étapes consistant à :

    - extraire une représentation du signal d'artefact de mouvement à partir de l'entrée de signal électrique biopotentiel (1, 2) en atténuant un signal de mode différentiel présent dans l'entrée de signal électrique biopotentiel (1, 2) à partir d'une première fréquence (F1) et en faisant passer une représentation du signal d'artefact de mouvement à partir de l'entrée de signal électrique biopotentiel (1, 2) jusqu'à une deuxième fréquence (F2) ;
    - soustraire la représentation extraite du signal d'artefact de mouvement à partir de l'entrée de signal électrique biopotentiel (1, 2).

**Figure 1**

# Figure 2a

# Figure 2b

## Figure 2c

# Figure 3

EP 3 939 495 B1

**Figure 4**

**Figure 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013116577 A

**Non-patent literature cited in the description**

- **SUNYOUNG KIM et al.** Real Time Digitally Assisted Analog Motion Artifact Reduction in Ambulatory ECG Monitoring System. *34th Annual International Conference of the IEEE EMBS, San Diego, California USA*, 28 August 2012

- **LI-MING BAI et al.** Using an Adaptive Filter to Remove ECG Motion Artifact Interference. *2018 IEEE International Conference on Consumer Electronics-Taiwan (ICCE-TW)*